# EUROPÄISCHE PATENTSCHRIFT

(11) **EP 3 750 574 B1**
(45) Veröffentlichungstag und Bekanntmachung des Hinweises auf die Patenterteilung: **03.01.2024**
(21) Anmeldenummer: 19180089.5
(22) Anmeldetag: 13.06.2019
(51) Int. Cl.: A61J 1/10, A61J 1/18, A61J 1/20, A61M 5/162

(54) **FLÜSSIGKEITSTRANSFERSYSTEM UND KOMPONENTEN DAFÜR**
LIQUID TRANSFER SYSTEM AND COMPONENTS FOR SAME
SYSTÈME DE TRANSFERT DE LIQUIDE ET COMPOSANTS ASSOCIÉS

(43) Veröffentlichungstag der Anmeldung: 16.12.2020
(73) Patentinhaber: Trenta2 S.r.l., 39100 Bozen (IT)
(72) Erfinder: GALLMETZER, Dietrich, 39018 Terlan (IT)
(74) Vertreter: Tergau & Walkenhorst Intellectual Property GmbH

(56) Entgegenhaltungen:
- WO-A1-2014/122643
- WO-A1-2016/042544
- WO-A2-2008/129550
- US-A1- 2008 277 021

## Beschreibung

Die Erfindung betrifft ein Flüssigkeitstransfersystem zum Transfer von medizinischen Substanzen oder Arzneimitteln. Sie bezieht sich weiter auf Komponenten und Bauteile eines solchen Transfersystems, insbesondere dessen Verbindungskomponenten und die mit solchen Verbindungskomponenten ausgerüsteten Bauteile wie Spritzen, Container (Phiolen) oder Applikationssysteme.

In modernen medizinischen Verfahren oder Therapien können Medikamente oder Substanzen zum Einsatz kommen, die an sich eigentlich toxisch oder auf sonstige Weise schädlich oder gefährlich sind. Für das mit der Handhabung solcher Substanzen betraute Personal, wie beispielsweise Apotheker und Krankenschwestern, können somit akute und langfristige Gesundheitsrisiken entstehen, gerade wenn es wiederholt Medikamenten oder Lösungsmitteln ausgesetzt ist, die während der Zubereitung, der Verabreichung von Medikamenten und anderen ähnlichen Behandlungen in die Luft entweichen könnten. Dieses Problem kann besonders schwerwiegend sein, wenn es sich um Zytotoxine, antivirale Medikamente, Antibiotika oder Radiopharmazeutika handelt. Die durch die Exposition gegenüber diesen Medikamenten potentiell entstehenden Gesundheitsrisiken umfassen ein erhöhtes Krebsrisiko, genetische Veränderungen und dergleichen. Des Weiteren können auch Probe- oder Blutabnahmen bei viralen Infektionen oder dergleichen ein erhöhtes Risiko für das handhabende Personal bedingen.

Bei der Durchführung von Infusionen ist es des Weiteren oft notwendig, ein Medikament oder einen Wirkstoff in die Infusionsflüssigkeit, in einen Infusionsbeutel oder in einen anderen Infusionsbehälter zu injizieren. Dies geschieht oft durch das Durchstoßen eines Septums oder einer anderen Flüssigkeitsbarriere einer Injektionsöffnung am Infusionsbeutel oder an der Infusionsleitung mit der Nadel einer mit der betreffenden medizinischen Flüssigkeit gefüllten Spritze. Doch schon vor diesem Schritt kann es notwendig sein, die medizinische Flüssigkeit aus einem Fläschchen oder einer Phiole in eine Spritze und dann von der Spritze in einen Sekundärbehälter zu transferieren. In jedem dieser Schritte kann das Personal durch Kontamination mit der medizinischen Flüssigkeit in Berührung kommen. Diese Kontamination kann verdampfte medizinische Flüssigkeit oder Aerosol in der Luft sein. Die Verunreinigungen können das Personal durch die Lunge oder durch verdampftes medizinisches Fluid oder Aerosol in der Luft kontaminieren, das auf der Haut kondensiert und danach in die Haut der betroffenen Person eindringt. Einige Medikamente sind sogar dafür bekannt, dass sie in Schutzhandschuhe eindringen und dadurch das Personal verunreinigen.

Einerseits aus den genannten Gründen des Gesundheitsschutzes, andererseits aber auch im Hinblick auf den Umstand, dass in jüngster Zeit Medikamente mit einem äußerst hohen Dosispreis zugelassen worden sind, ist es dringend wünschenswert oder sogar notwendig, die Abgabe auch kleinster Mengen solcher Medikamente oder Wirkstoffe an die Umgebung zuverlässig zu vermeiden. Zu diesem Zweck sind so genannte geschlossene Transfersysteme gebräuchlich, bei denen in der Art einer gekapselten Ausführung in jeder Phase des Flüssigkeitstransfers sichergestellt ist, dass der Wirkstoff oder die von diesem freigesetzten Gase oder Aerosole nicht in die Umgebung entweichen können.

Derartige Flüssigkeitstransfersysteme umfassen üblicherweise eine erste Verbindungskomponente oder einen ersten Adapter zum Anschluss an einen ersten Fluidbehälter, wie beispielsweise eine Spritze, und eine zweite Verbindungskomponente oder einen zweiten Adapter zum Anschluss an einen Wirkstoffbehälter, eine Phiole, eine zweite Spritze oder eine intravenöse Leitung, die einen Flüssigkeitszugang zum Kreislauf des Patienten bereitstellt. Dabei kann der Arzt oder die behandelnde Person beispielsweise eine Spritze über den Spritzenadapter und den zugeordneten Phiolen Adapter mit einer Phiole verbinden und das darin befindliche Medikament in die Spritze einsaugen. Anschließend werden die Adapter getrennt, und die Spritze wird über den Spritzenadapter und einen zugeordneten Leitungsadapter mit einer intravenösen Leitung oder einem intravenösen Zugang verbunden, so dass das in der Spritze befindliche Medikament an den Patienten verabreicht werden kann.

Ein solches Flüssigkeitstransfersystem einschließlich der erforderlichen Komponenten wie Verbindungsadapter und dergleichen ist beispielsweise aus der WO2016199133A1 (oder aus der WO2016042544) bekannt.

Der Erfindung liegt die Aufgabe zugrunde, ein Flüssigkeitstransfersystem zum Transfer von medizinischen Substanzen oder Arzneimitteln der oben genannten Art, einschließlich seiner wesentlichen Komponenten wie insbesondere der Verbindungskomponenten, anzugeben, mit dem bei besonders einfach gehaltener Handhabung die unbeabsichtigte Abgabe oder Freisetzung von Wirkstoffbestandteilen oder Gasen oder Aerosolen hiervon besonders zuverlässig vermieden ist. Bezüglich einer ersten Verbindungskomponente, insbesondere für eine medizinischen Spritze, wird diese Aufgabe erfindungsgemäß gelöst mit einem eine Anzahl von Hohlnadeln umfassenden, in einem rohrförmigen, von einem Flanschmantel umgebenen Verbindungsstutzen angeordneten Nadelsystem und mit einer innerhalb des Verbindungsstutzens angeordneten, die distale(n) Spitze(n) der oder jeder Nadel des Nadelsystems im unbelasteten Zustand dichtend umschließenden elastischen Nadelkappe, wobei der Flanschmantel um das Nadelsystem und um die dieses umschließende Nadelkappe rotierbar ist und mit einem Gewinde zum Anschluss an eine zweite Verbindungskomponente versehen ist.

Vorteilhafte Ausgestaltungen der Erfindung sowie auch als eigenständig erfinderisch angesehene weitere Aspekte oder Varianten sind Gegenstand der Unteransprüche und/oder der nachfolgenden Figurenbeschreibung entnehmbar.

Die Erfindung geht von der Überlegung aus, dass für einen sicheren und verlustfreien Flüssigkeitstransfer zwischen Spritze und weiterem Behältnis, beispielsweise einer Phiole, eine stabile und dichtende Verbindung zwischen den jeweiligen Verbindungskomponenten besonders bedeutsam ist. Dazu muss einerseits eine geeignete Dichtung an den Grenz- oder Kontaktflächen beider Verbindungskomponenten bereitgestellt werden, und andererseits sollte ein geeigneter Kraftschluss zwischen den Verbindungskomponenten, zum einen zum Zweck der mechanischen Stabilisierung der Komponenten relativ zueinander und zum anderen zum Zweck einer Verbesserung der Dichtwirkung durch Anpressen, hergestellt werden. Bei den bekannten Systemen, beispielsweise dem aus der WO2016199133A1 bekannten, ist hierzu eine Verhakung oder Verrastung der Verbindungskomponenten miteinander vorgesehen, wobei geeignet positionierte Schwenkarme die Verrastung der Komponenten bewirken.

Abweichend von diesem Auslegungsprinzip und im Hinblick auf eine besonders gute Dosierbarkeit der aufgewandten Haltekräfte ist nunmehr eine Verschraubung der Verbindungskomponenten miteinander vorgesehen, wobei die Haltekraft zwischen den Verbindungselementen durch geeignete Betätigung der Verschraubung modifiziert und bedarfsgerecht eingestellt werden kann. Ergänzend hierzu ist als Versiegelung und abdichtender Abschluss für die als eigentliche Transferkanäle in der Verbindungskomponente vorgesehenen Hohlnadeln eine Nadelkappe aus vergleichsweise weichem, elastischem Material vorgesehen. Diese wird bei Kontakt mit dem anderen Verbindungselement verformt und zurückgedrängt, so dass die in Inneren befindlichen Hohlnadeln sie durchstoßen und damit zur Herstellung der medienseitigen Verbindung nach außen freigelegt werden. Je nach Einstellung der Verschraubung kann die Verformung der Nadelkappe dabei unterschiedlich sein, so dass entsprechend auch die durch die Nadelkappe und deren Kontakt mit der Gegenfläche gegebene Dichtwirkung geeignet optimiert werden kann. Nach Abschluss des Flüssigkeitstransfers und nach der Trennung der Komponenten nimmt die Nadelkappe aufgrund der Elastizität ihres Grundmaterials jedoch wieder ihre ursprüngliche Form ein und umschließt somit die Nadelspitzen wieder, so dass auch nach Abschluss des Transfers eine zuverlässige Umhüllung und Versiegelung des Spritzeninneren einschließlich der Nadelauslässe gewährleistet ist.

Ganz besonders bevorzugt ist die Nadelkappe dabei Material mit sehr guter Druckverformungsresistenz und besonders guten Druckrelaxations-Eigenschaften ausgeführt. Ganz besonders bervorzugt ist dafür ein thermoplastisches Elastomer (TPE) vorgesehen.

In ganz besonders vorteilhafter und als eigenständig erfinderisch angesehener Ausgestaltung ist dabei innerhalb der Nadelkappe ein Federelement, besonders bevorzugt eine Spiralfeder, angeordnet, das nach Abschluss des Transfers die Wiederherstellung der ursprünglichen Form der Nadelkappe unterstützt. Dabei kann beispielsweise eine Spiralfeder, das Nadelsystem umgebend, innerhalb der Nadelkappe angeordnet sein, die sich einerseits an einer Bodenplatte, in der die Nadeln des Nadelsystems gehalten sind, und andererseits an der Deckelfläche der Nadelkappe abstützt. Bei der Verformung der Nadelkappe infolge des Kontakts mit dem anderen Verbindungselement wird das Federelement zusammen mit der Nadelkappe mit verformt und dabei vorgespannt. Nach Abschluss des Flüssigkeitstransfers und nach der Trennung der Komponenten entspannt sich das Federelement wieder und unterstützt damit die erwünschte Rückverformung der Nadelkappe, bis diese wieder ihre ursprüngliche, die freilegenden Spitzen des Nadelsystems vollständig umschließende Form einnimmt.

Um einen vollständig gekapselten Flüssigkeitstransfer zu ermöglichen, bei dem weder Flüssigkeitsverluste noch ein Entweichen von Gasen oder Aerosolen aus der Flüssigkeit an die Umgebung in Kauf genommen werden müssen, ist das Nadelsystem vorteilhafterweise als Doppelnadelsystem ausgeführt. Dieses umfasst, analog zu der aus der WO2016199133A1 bekannten Ausgestaltung, eine erste, für den eigentlichen Flüssigkeitstransfer vorgesehene erste Hohlnadel und eine zweite, für einen Gastransfer vorgesehene zweite Hohlnadel.

Um eine besonders zuverlässige und sichere medienseitige Verbindung mittels des Nadelsystems zu ermöglichen, ist erfindungsgemäß der Flanschmantel frei um das Nadelsystem und um die dieses umschließende Nadelkappe herum rotierbar ausgeführt. Damit ist die Nutzung des Verbindungsgewindes, mit dem die mechanisch vergleichsweise stabile und damit besonders günstige Verschraubung der Verbindungskomponenten miteinander bewirkt werden soll, auf besonders einfache und günstige Weise und insbesondere ohne Störung oder Beeinträchtigung der medienseitigen Verbindung über das Nadelsystem möglich. Dies kann in besonders vorteilhafter Weise genutzt werden, um die Nadeln des Nadelsystems hinsichtlich ihrer Dimensionierung besonders fein, insbesondere vergleichsweise dünnwandig und mit vergleichsweise gering gehaltenem Außendurchmesser, ausführen zu können.

Bezüglich einer zweiten Verbindungskomponente, insbesondere für einen Container für medizinische Substanzen oder Arzneimittel oder für ein Transfersystem für medizinische Substanzen oder Arzneimittel, und insbesondere zum Anschluss an die vorstehend beschriebene erste Verbindungskomponente, wird die vorstehend genannte Aufgabe in als eigenständig erfinderisch angesehener Ausführung gelöst mit einem mit einem zum Anschluss an dem Gewinde des Flanschmantels der ersten Verbindungskomponente vorgesehenen Gewinde versehenen Verbindungsstutzen, in dem ein aus einem Polypropylen, bevorzugt aus PP Purell HP570, bestehender Verschlussstopfen angeordnet ist.

Im Gegensatz zu üblichen Systemen, bei denen beispielsweise Container für medizinische Substanzen oder Arzneimittel mit einem siegelartigen Verschlussdeckel aus Aluminium oder dergleichen verschlossen sind, kann durch eine solche zweite Verbindungskomponente der Container wiederverschließbar ausgeführt sein. Bei den herkömmlichen Systemen wird nämlich bei der erstmaligen Verwendung der siegelartige Verschlussdeckel durch das Nadelsystem durchstochen, so dass er nach erfolgtem Flüssigkeitstransfer und der Trennung der Komponenten offen bleibt, so dass die im Container befindliche Substanz vollständig transferiert werden muss; eventuell im Container verbleibende Restbestände oder auch Gase oder Aerosole müssen dann gesondert und in einem sicheren und gekapselten System entsorgt werden. Im vorliegenden, als erfinderisch angesehenen System führt hingegen die Ausführung des Verschlussstopfens dazu, dass das Material nach Entfernung des Nadelsystems wieder seine ursprüngliche Position einnimmt und den Container somit erneut dichtend verschließt. Somit ist in gewissem Umfang eine Mehrfachnutzung des Containers und damit eine chargenweise Entnahme des darin befindlichen Wirkstoffs möglich.

Zur Herstellung der vorgesehenen Schraubverbindung der Komponenten miteinander ist das Gewinde der zweiten Verbindungskomponente vorteilhafterweise hinsichtlich Auslegung, Dimensionierung und dergleichen an das Gewinde der ersten Verbindungskomponente angepasst. Ganz besonders vorteilhaft bilden die genannten Komponenten dabei ein Luer-Gewinde.

Als eigenständig erfinderisch angesehen wird ebenfalls ein mit einer solchen zweiten Verbindungskomponente ausgerüsteter Container für medizinische Substanzen oder Arzneimittel. In besonders vorteilhafter Ausgestaltung ist dieser mit einer äußeren, die Verbindungskomponente umgebenden Kappe versehen, die mit einem Einwegverschluss versehen ist. Dieser Einwegverschluss, der beispielsweise einen abreissbaren oder verplombt ausgeführten Siegeldeckel umfassen kann, erlaubt eine problemlose und zuverlässige Identifikation, ob der Container bereits zum Flüssigkeitstransfer verwendet wurde oder nicht, und erleichtert somit die Zuordnung, ob der Container bereits "angebrochen" ist und somit bevorzugt für eine weitere Flüssigkeitsentnahme verwendet werden sollte, bis er vollständig entleert ist und somit entsorgt werden sollte.

Die Erfindung betrifft weiterhin in als eigenständig erfinderisch angesehener Ausführung eine medizinische Spritze, die an ihrem distalen Ende mit einer Verbindungskomponente nach einer der vorstehend beschriebenen Ausführungen versehen ist. Des Weiteren betrifft die Erfindung in ebenfalls als eigenständig erfinderisch angesehener Ausgestaltung ein Flüssigkeitstransfersystem zum Transfer von medizinischen Substanzen oder Arzneimitteln mit einer ersten Verbindungskomponente der vorstehend beschriebenen Ausgestaltung und mit einer, hinsichtlich der Dimensionierung und Ausführung der Verbindungstutzen an diese angepasst, einer zweiten Verbindungskomponente der vorstehend beschriebenen Ausgestaltung.

Ein Ausführungsbeispiel der Erfindung wird anhand einer Zeichnung näher erläutert. Darin zeigen:
- Fig. 1: eine über Verbindungskomponenten eines Flüssigkeitstransfersystems mit einem Container für Arzneimittel oder Medikamente verbindbare medizinische Spritze im Längsschnitt,
- Fig. 2: die Komponenten gem. Fig. 1 im Längsschnitt im verbundenen Zustand,
- Fig. 3: einen vergrößerten Ausschnitt der Komponenten nach Fig. 2,
- Fig. 4: eine besonders bevorzugte Ausführungsform eines Containers zur Verwendung im Flüssigkeitstransfersystem nach Fig. 1,
- Fig. 5: im Längsschnitt eine besonders bevorzugte Ausführungsform einer ersten Verbindungskomponente zur Verwendung im Flüssigkeitstransfersystem nach Fig. 1, im Zustand vor der Verbindung der Komponenten miteinander,
- Fig. 6: die Verbindungskomponente gem. Fig. 5 im Längsschnitt bei miteinander verbundenen Komponenten,
- Fig. 7: einen Spritzenadapter vor der Montage mit einer zugeordneten Spritze im Längsschnitt,
- Fig. 8: die Spritze gem. Fig. 7 mit montiertem Spritzenadapter,
- Fig. 9: einen Infusionsbeutel mit Adapter,
- Fig. 10: den mit Adapter versehenen Infusionsbeutel nach Fig. 9 vor der Montage mit einer zugeordneten Spritze im Längsschnitt, und
- Fig. 11: die Spritze gem. Fig. 10 mit montiertem Infusionsbeutel.

Gleiche Teile sind in allen Figuren mit denselben Bezugszeichen versehen. Die Erfindung wird nachfolgend vorwiegend anhand des Ausführungsbeispiels erläutert, bei dem eine medizinische Spritze mit einer ersten Verbindungskomponente der genannten Art und ein Container für medizinische Substanzen oder Arzneimittel mit einer zweiten Verbindungskomponente der beschriebenen Art ausgerüstet ist. Selbstverständlich sind aber auch andere Ausführungen denkbar und von der vorliegenden Erfindung umfasst, bei der andere Bestandteile eines medizinischen Systems, beispielsweise eine Zugangsschnittstelle zu einem intravenösen Applikationssystem oder einen Adapter für einen Infusionsbeutel ("IV-Bag"), mit einer solchen zweiten Verbindungskomponente, oder auch eine "inverse Anordnung", bei der eine medizinische Spritze mit der zweiten Verbindungskomponente und das jeweils andere Bauteil, also beispielsweise ein Container oder eine Zugangsschnittstelle, mit einer solchen ersten Verbindungskomponente versehen ist. Ebenso sind selbstverständlich sind aber auch andere Ausführungen im nicht-medizinischen Bereich denkbar und von der vorliegenden Erfindung umfasst, bei denen unter zuverlässigem Gas- oder Materialabschluss Flüssigkeitstransfers vorgenommen werden müssen, ohne dass die handhabenden Personen einer Exposition ausgesetzt sind, beispielsweise bei Anwendungen in der Verfahrenstechnik oder für gefährliche chemische Prozesse und dergleichen.

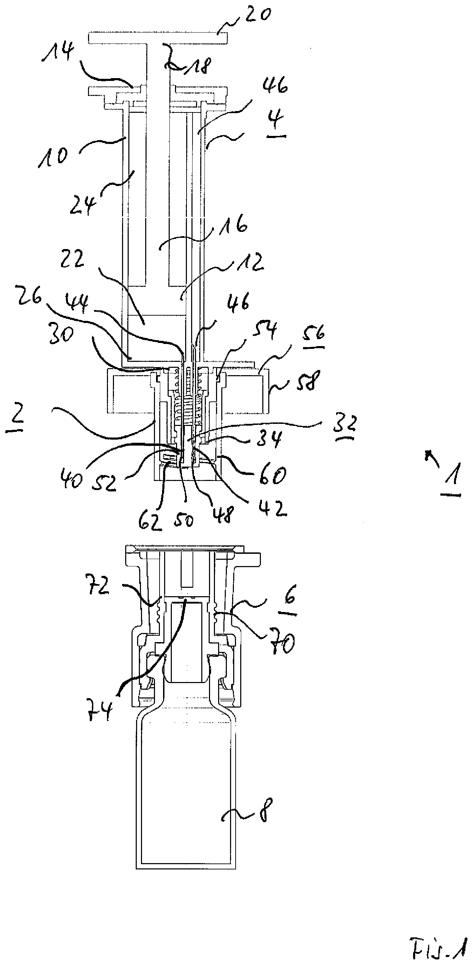

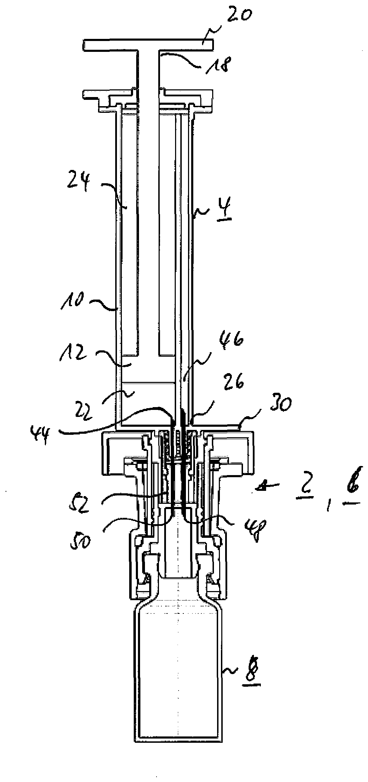

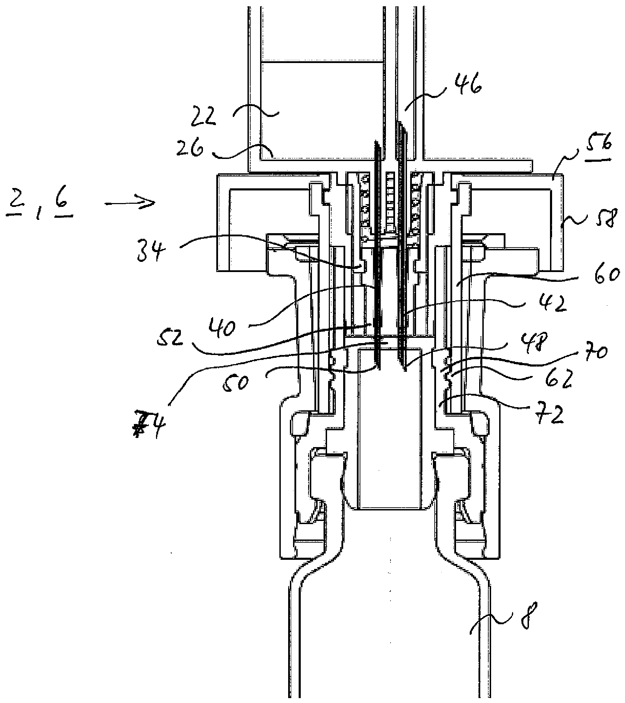

Das erfindungsgemäße Flüssigkeitstransfersystem 1 wird nachfolgend somit anhand einer mit einer ersten Verbindungskomponente 2 versehenen medizinischen Spritze 4 und eines mit einer zweiten Verbindungskomponente 6 versehenen Containers 8 für medizinische Substanzen oder Arzneimittel erläutert. In Fig. 1 sind dabei die Komponenten, also die Spritze 4 und der Container 8 im Längsschnitt im separaten, noch getrennten Zustand gezeigt; Figs. 2 und 3 zeigen die Komponenten analog (Fig 3 ausschnittsweise) im Längsschnitt nach der Herstellung der Verbindung miteinander.

Die Spritze 4 umfasst einen entsprechend einer herkömmlichen Bauweise zylindrisch oder rohrförmig ausgeführten, ein Spritzengehäuse bildenden Hohlkörper 10, in dem ein in seinen Außenabmessungen passgenau an die Innenkontur des Hohlkörpers 10 angepasster Kolben 12 verschiebbar geführt ist. Der Kolben 12 ist endseitig an einem dichtend durch eine Deckelplatte 14 geführten Betätigungsstö-ßel 16 angeordnet, der seinerseits an seinem freien Ende 18 mit einer Drückerplatte 20 versehen ist. Der Kolben 12 teilt das Innenvolumen des Hohlkörpers 10 in ein distales, für die Aufnahme von Flüssigkeit vorgesehenes Teilvolumen 22 und ein proximales, für die Aufnahme von Gasen oder Aerosolen vorgesehenes Teilvolumen 24, deren Volumeninhalte durch die Positionierung des Kolbens 12 innerhalb des Hohlkörpers 10 veränderbar sind. Die erste Verbindungskomponente 2 ist dabei am distalen Ende 26 des Hohlkörpers 10 und damit des distalen Teilvolumens 22 angeordnet.

Die als eigenständig erfinderisch angesehene erste Verbindungskomponente 2, die im Ausführungsbeispiel integral mit dem Hohlkörper 10 ausgeführt, aber auch als separate, nachrüstbare Komponente ausgeführt sein könnte, umfasst eine distale Endplatte 30. die das distale Teilvolumen 22 des Hohlkörpers dichtend abschließt. Die Endplatte 30 bildet einen Nadelhalter für ein Nadelsystem 32 und auf ihrer vom Teilvolumen 22 abgewandten Seite einen das Nadelsystem 32 umgebenden, rohrförmig ausgestalteten Verbindungsstutzen 34 zur Herstellung einer Verbindung mit der zweiten Verbindungskomponente 6 aus.

Das Nadelsystem 32 ist im Ausführungsbeispiel als Doppelnadelsystem ausgeführt und umfasst eine erste, für den Flüssigkeitstransfer vorgesehenen Hohlnadel 40 und eine zweite, für einen Gastransfer vorgesehene Hohlnadel 42. Die erste Hohlnadel 40 ragt dabei mit ihrem inneren Ende 44 in das Teilvolumen 22 hinein und bildet somit einen Flüssigkeitskanal für den Flüssigkeitsaustausch. Die zweite Hohlnadel 42 hingegen ist innenseitig mit einem Gaskanal 46 verbunden, der in der Art eines Bypasses an der Innenwand des Hohlkörpers 10 entlang bis hinein in das proximale Teilvolumen 24 geführt ist. Über die zweite Hohlnadel 42 kann von deren distaler Nadelspitze 48 aus somit in der Art einer gekapselten Ausführung Gas oder Aerosol in das proximale Teilvolumen 24 überführt oder von diesem aus zur Nadelspitze 48 hin geführt werden. Analog zu der in der WO2016199133A1 beschriebenen Funktionsweise kann somit bei Anschluss des Containers 4 Flüssigkeit von diesem in das distale Teilvolumen 22 überführt und gleichzeitig ohne Abgabe von Substanzen nach außen und mit automatisch stattfindendem Druckausgleich Gas vom proximalen Teilvolumen 24 in den Container 4 überführt werden.

Die erste und die zweite Hohlnadel 40, 42 ragen mit ihren distalen Nadelspitzen 48, 50 aus dem offenen Ende des rohrförmigen Verbindungsstutzens 34 heraus. Um eine zuverlässige Kapselung des Systems zu gewährleisten, ist zudem innerhalb des Verbindungsstutzens 34 eine elastische, vergleichsweise leicht verformbare Nadelkappe 52 befestigt, die die distalen Spitzen 48, 50 der Hohlnadeln 40, 42 des Nadelsystems im unbelasteten Zustand dichtend umschließt. In der im Ausführungsbeispiel gezeigten besonders bevorzugten Ausführungsform besteht die Nadelkappe 52 dabei aus einem thermoplastischen Elastomer (TPE). Bei Herstellung einer Verbindung mit der zweiten Verbindungskomponente 6, insbesondere durch Andrücken an eine vorgesehene Kontaktplatte, verformt sich die Nadelkappe 52, wobei die distalen Spitzen 48, 50 die Nadelkappe 52 durchstoßen und somit frei liegen, so dass ein Medienaustausch ermöglicht wird.

Außenseitig ist der Verbindungsstutzen 34 von einer auf einer umlaufenden Rippe 54 gelagerten Verbindungsmuffe 56 umgeben. Diese ist um das Nadelsystem 32 rotierbar gelagert und weist einen Betätigungsrand 58, über den der Nutzer eine Rotation bewirken kann, und einen Flanschmantel 60 auf, der innenseitig mit einem Gewinde 62 versehen ist. Das Gewinde 62 in Kombination mit dem Flanschmantel 60 ist dabei im Ausführungsbeispiel als Komponente einer Luer-Verbindung ausgestaltet. Im Ausführungsbeispiel ist das Gewinde 62 dabei lediglich mit einem Gewindegang oder Gewindezug ausgeführt, kann aber selbstverständlich aber auch noch weitere Gewindegänge und/oder Gewindezüge aufweisen.

Der in Fig. 1 ebenfalls im Längsschnitt gezeigte Container 8 für medizinische Substanzen oder Arzneimittel, auch als Phiole bezeichnet, ist entsprechend mit einer zum Anschluss an die erste Verbindungskomponente 2 ausgestalteten zweiten Verbindungskomponente 6 versehen. Diese umfasst einen mit einem mit einem Gewinde 70 versehenen Verbindungsstutzen 72, in dem ein aus dem besonders bervorzugten und hinsichtlich der Materialwahl als eigenständig erfinderisch angesehenen Material PP Purell HP 570 bestehender Verschlusstopfen 74 angeordnet ist. Der mit dem Gewinde 70 versehene Verbindungsstutzen 72 ist dabei an den Flanschmantel 60 und dessen Gewinde 62 angepasst ausgeführt, so dass diese Komponenten miteinander eine Luer-Verbindung bilden.

Zur Herstellung der medienseitigen Verbindung der Verbindungskomponenten 2, 6 miteinander, wie sie in der Gesamtschau in Fig. 2 und in ausschnittsweiser Vergrößerung in Fig. 3 gezeigt ist, wird die mit der ersten Verbindungskomponente 2 versehene Spritze 4 zunächst derart mit dem mit der zweiten Verbindungskomponente 6 versehenen Container 8 in Kontakt gebracht, dass das Gewinde 62 des Flanschmantels 60 mit dem Gewinde 70 des Verbindungstutzens 72 in Eingriff kommt. Dadurch entsteht eine Schraubverbindung zwischen diesen Komponenten, die durch Rotation des Flanschmantels 60, also die Betätigung des Betätigungsrands 58, weiter angezogen werden kann. Hierdurch wird das erste Verbindungselement 2 weiter in Längsrichtung der Spritze 4 gesehen in Richtung zur zweiten Verbindungskomponente 6 und somit den Container 8 hin bewegt. Sobald die Endfläche der Nadelkappe 52 dabei an den als Kontaktplatte dienenden Verschlussstopfen 74 anschlägt, beginnt sich die Nadelkappe 52 aufgrund ihrer elastischen Ausgestaltung zu verformen, und die distalen Nadelspitzen 48, 50 beginnen die Nadelkappe 52 zu durchdringen. Bei weiterem Anziehen der Schraubverbindung durchstoßen die distalen Nadelspitzen 48, 50 die Nadelkappe 52 vollständig und beginnen den Verschlussstopfen 74 zu durchdringen.

Bei weiterer Betätigung der Schraubverbindung bis hin zu der in Fig. 2 und 3 gezeigten Endposition durchstoßen die distalen Nadelspitzen 48, 50 auch den Verschlussstopfen 74 vollständig. In diesem Zustand bildet die erste Hohlnadel 40 einen offenen Flüssigkeitskanal und ermöglicht den Flüssigkeitsaustausch zwischen dem Innenraum des Containers 8 und dem distalen Teilvolumen 22, und über die zweite Hohlnadel 42 und den an diese angeschlossenen Gaskanal 46 ist ein Gasaustausch zwischen dem proximalen Teilvolumen 24 und dem Innenraum des Containers 8 ermöglicht. Zum verlustfreien und gekapselten Flüssigkeitstransfer vom Container 8 in das distale Teilvolumen 22 der Spritze 4 kann nun, ausgehend von einem Anfangszustand, in dem der Kolben 12 unmittelbar benachbart zum distalen Ende 26 des Hohlkörpers 10 positioniert ist, der Kolben 12 innerhalb des Hohlkörpers 10 von dessen distalem Ende 26 weg bewegt werden. Dabei strömt Flüssigkeit vom Inneren des Containers 8 in das distale Teilvolumen 22 der Spritze 4 über. Gleichzeitig wird das im proximalen Teilvolumen 24 befindliche Gas komprimiert, so dass es zum Druckausgleich über den Gaskanal 46 und die zweite Hohlnadel 42 in den Innenraum des Containers 8 strömt. Damit ist ein sicherer und gekapselter Medienaustausch zwischen Spritze 4 und Container 8 ermöglicht, ohne dass Flüssigkeit oder Gas an die Umgebung austritt.

Nach Entnahme der vorgesehenen Flüssigkeitsmenge aus dem Container 8 werden die Komponenten durch Lösen der Schraubverbindung wieder getrennt. Dabei werden die Hohlnadeln 40, 42 des Nadelsystems 32 in Längsrichtung der Spritze 4 gesehen wieder aus dem Innenraum des Containers 8 herausgezogen. Sobald die Nadelspitzen 48, 50 dabei vollständig aus dem Verschlussstopfen 74 herausgezogen wurden, nimmt dieser aufgrund der Materialwahl wieder seine ursprüngliche Form ein und dichtet dabei den Innenraum des Containers 8 nach außen hin wieder ab. Sobald die Endfläche der Nadelkappe 52 zudem von dem als Kontaktplatte dienenden Verschlussstopfen 74 abgehoben wird, beginnt die Nadelkappe 52 aufgrund ihrer Materialeigenschaften, ihre ursprüngliche Form wieder einzunehmen, so dass die Nadelspitzen 48, 50 wieder vollständig in ihrem Innenraum positioniert werden und somit die Nadelkappe 52 die Nadelspitzen 48, 50 wieder vollständig umschließt. Damit ist nach der Trennung der Komponenten voneinander wieder eine vollständige Kapselung des Innenraums des Containers 8 einerseits und des Innenraums des Hohlkörpers 10 gegeben.

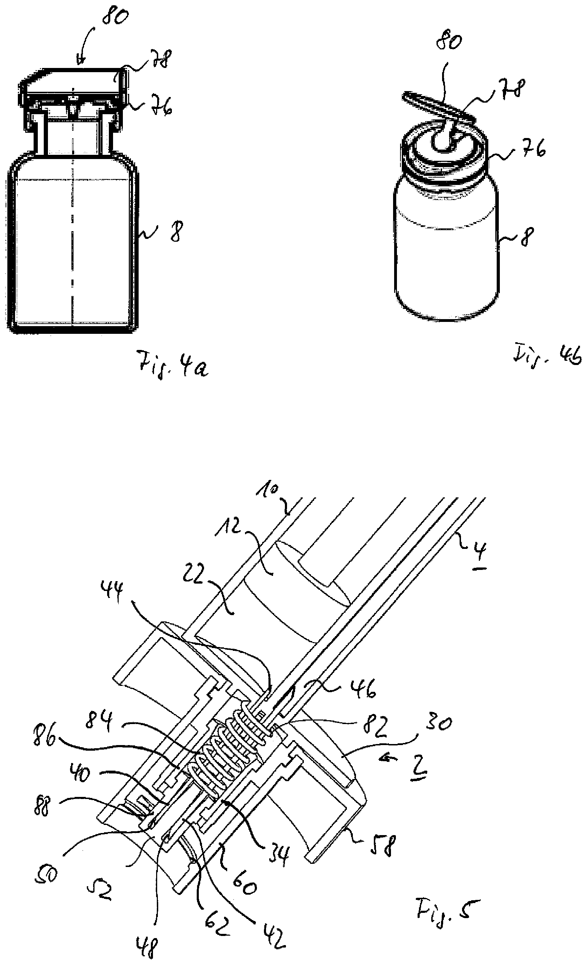

Eine besonders bevorzugte und als eigenständig erfinderisch angesehene Ausgestaltung der Phiole oder des Containers 8 ist im Längsschnitt in Fig. 4 gezeigt. Wie der seitlichen Ansicht in Fig. 4a und auch der Darstellung des montierten Zustands in perespektivischer Ansicht in Fig. 4b entnehmbar ist, umfasst der Container 8 in dieser Ausführungsform eine äußere, die Verbindungskomponente 6 umgebende Verschlusskappe 76, die mit einem Einwegverschluss 78 versehen ist. Der Einwegverschluss 78 umfasst im Ausführungsbeispiel einen abreissbaren, oder alternativ oder zusätzlich beispielsweise einen verplombt ausgeführten, Siegeldeckel 80, der beim erstmaligen Öffnen des Containers 8 entfernt wird. Diese Ausgestaltung erlaubt eine problemlose und zuverlässige Identifikation, ob der Container 8 bereits zum Flüssigkeitstransfer verwendet wurde oder nicht, und erleichtert somit die Zuordnung, ob der Container 8 bereits "angebrochen" ist und somit bevorzugt für eine weitere Flüssigkeitsentnahme verwendet werden sollte, bis er vollständig entleert ist und somit entsorgt werden sollte.

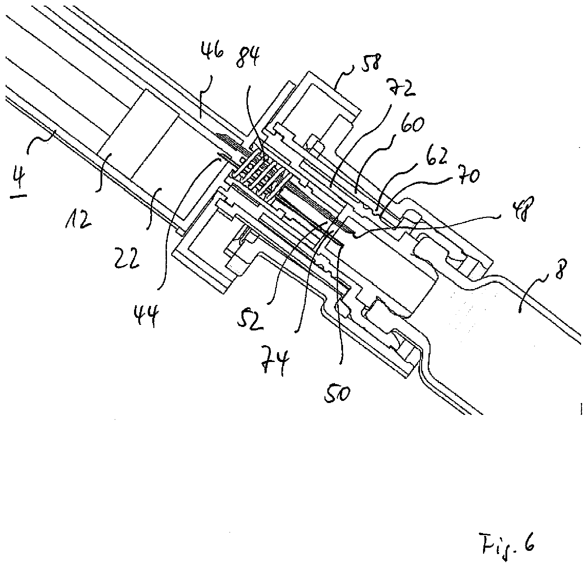

Eine ganz besonders vorteilhafte und als eigenständig erfinderisch angesehene Ausgestaltung der ersten Verbindungskomponente 2 ist im Längsschnitt in vergrößertem Ausschnitt in Fig. 5 (Zustand vor der Verbindung der Komponenten miteinander) und in Fig. 6 (Zustand bei miteinander verbundenen Komponenten) gezeigt. Wie dieser Darstellung entnehmbar ist, ist in dieser Ausführungsform als weiteres Bauteil innerhalb der Nadelkappe 52 ein Federelement 82, im Ausführungsbeispiel eine Spiralfeder 84, angeordnet. Die Spiralfeder 84 ist dabei im Ausführungsbeispiel, das Nadelsystem 32 umgebend, innerhalb der Nadelkappe 52 angeordnet. Sie stützt sich einerseits an der Deckelplatte 30 des distalen Teilvolumen 22, in der die Nadeln 40, 42 des Nadelsystems 32 gehalten sind, und andererseits an der Deckelfläche der Nadelkappe 52 ab. Im dargestellten Ausführungsbeispiel ist zudem die Nadelkappe 52 zweiteilig ausgeführt und umfasst zusätzlich zum eigentlichen Kappenkörper 86 einen distal an diesem angebrachten Nadelschutz 88, wobei sich in dieser Ausführungsform die Spiralfeder 84 am Boden des Nadelschutzes 88 abstützt.

Im in Fig. 5 gezeigten Zustand vor der Verbindung der Komponenten miteinander ist die Spiralfeder 84 entspannt. Als Folge der Verformung der Nadelkappe 52 infolge des Kontakts mit dem anderen Verbindungselement 6 wird das Federelement 82 jedoch zusammen mit der Nadelkappe 52 mit verformt und dabei vorgespannt; dieser Zustand ist in Fig. 5 gezeigt. Nach Abschluss des Flüssigkeitstransfers und nach der Trennung der Komponenten entspannt sich das Federelement 82 wieder und unterstützt damit die erwünschte Rückverformung der Nadelkappe 52, bis diese wieder ihre ursprüngliche, die freilegenden Spitzen 48,50 des Nadelsystems 32 vollständig umschließende Form einnimmt.

Die vorstehend beschriebene, mit der ersten Verbindungskomponente 2 ausgerüstete medizinische Spritze 4 kann beispielsweise zum Flüssigkeitstransfer zwischen verschiedenen Containern vom Typus des Containers 8 oder auch zum Transfer von Flüssigkeiten aus einem solchen Container 8 in einen Intravenös (IV) - Zugang benutzt werden. In solchen Anwendungsfällen wird die medizinische Spritze 4 jeweils über ihre Verbindungskomponente 2 mit der zugeordneten zweiten Verbindungskomponente 6 des jeweiligen Containers 8 bzw. einer Zugangsschnittstelle des Intravenös-Zugangs verbunden, so dass Flüssigkeit vom jeweiligen Container 8 in das distale Teilvolumen 22 der Spritze 4 eingezogen oder von diesem in einen entsprechenden Container 8 oder den IV-Zugang ausgebracht werden kann.

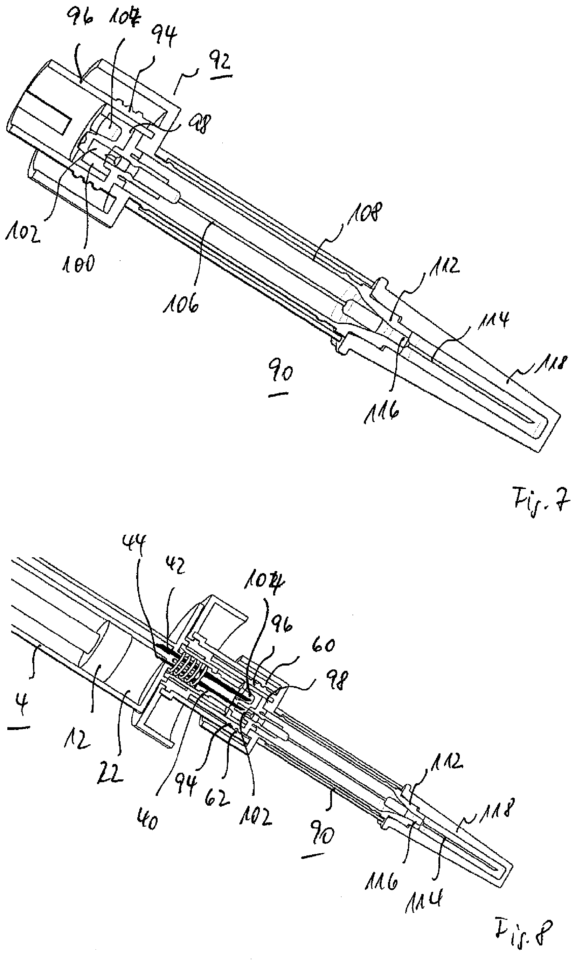

Alternativ und in einer als eigenständig erfinderisch angesehenen Ausführungsform kann die Spritze 4 nach der Befüllung mit der als Medikament oder Wirkstoff transferierten Flüssigkeit aber auch zur direkten Injektion der Flüssigkeit und somit als Injektionsspritze hergerichtet werden. Hierzu ist ein als eigenständig erfinderisch angesehener Spritzenadapter 90 vorgesehen, wie er - jeweils im Längsschnitt gemeinsam mit dem Endbereich der Spritze 4 - in Fig. 7 vor und in Fig. 8 nach der Montage mit der Spritze 4 gezeigt ist.

Der in den Figs. 7, 8 im Längsschnitt gezeigte Spritzenadapter 90 ist wie dargestellt zur Montage an die medizinische Spritze 4 unter Nutzung von deren erster Verbindungskomponente 2 ausgelegt. Dazu ist der Spritzenadapter 90 entsprechend mit einer zum Anschluss an die erste Verbindungskomponente 2 geeignet ausgestalteten weiteren Verbindungskomponente 92 versehen. Diese umfasst analog zu den vorstehend beschriebenen Varianten einen mit einem Gewinde 94 versehenen Verbindungsstutzen 96. Dieser ist dabei - ebenso wie die entsprechenden Komponenten der zweiten Verbindungskomponente 6 - an den Flanschmantel 60 der ersten Verbindungskomponente 2 und dessen Gewinde 62 angepasst ausgeführt, so dass diese Komponenten miteinander eine Luer-Verbindung bilden.

Innerhalb des Verbindungsstutzens 96 ist ein Kontaktelement 98 zur Herstellung einer medienseitigen Verbindung mit dem Nadelsystem 32 angeordnet. Das Kontaktelement 98 wird im Wesentlichen durch einen stopfenartig ausgeführten Grundkörper 100 gebildet, der eine Kavität oder einen Hohlraum 102 zur Aufnahme der Nadelspitze 50 der ersten, für den Flüssigkeitstransfer vorgesehenen Hohlnadel 40 und eine weitere Kavität oder einen weiteren Hohlraum 104 zur Aufnahme der Nadelspitze 48 der zweiten, medienseitig mit dem Gaskanal 46 verbundenen Hohlnadel 42 aufweist. Ein gekapselter Gasaustausch ist in diesem Falle nicht vorgesehen, und dementsprechend ist der weitere Hohlraum 104 in der Art eines Sacklochs ausgeführt und mit keinen weiteren Medienkanälen verbunden.

Im Gegensatz dazu ist der Hohlraum 102 als Transferraum zur Weiterleitung der über die Hohlnadel 40 aus dem distalen Teilvolumen 22 der Spritze 4 eingespeisten Flüssigkeit ausgelegt und vorgesehen. Dazu mündet in den Hohlraum 102 das proximale Ende einer Transfernadel 106. Diese ist innerhalb eines zylindrisch oder rohrförmig ausgeführten, das Gehäuse des Spritzenadapters 90 bildenden Hohlkörpers 108 angeordnet. Am vorderen oder distalen Ende 110 des Hohlkörpers 108 ist ein Nadelhalter 112 befestigt, in dem die zur Injektion des Wirkstoffs vorgesehene Hohlnadel 114 in einer Lagermuffe 116 gelagert ist. Der Nadelhalter 112 könnte dabei einstückig mit dem das Gehäuse bildenden Hohlkörper 108 ausgeführt sein. Im Ausführungsbeispiel ist der Nadelhalter 112 aber in als eigenständig erfinderisch angesehener Ausführung als separates Bauteil ausgestaltet. Der Nadelhalter 112 ist dabei vorliegend auf den das Gehäuse bildenden Hohlkörper 108 aufgesteckt oder aufsteckbar, könnte aber auch mittels eines Gewindes, beispielsweise eines Luer-Gewindes, anschraubbar ausgeführt sein. Der Hohlkörper 108 ist außen von einer in Längsrichtung verschiebbaren Nadelschutzhülse 118 umgeben.

In weiterer alternativer und ebenfalls als eigenständig erfinderisch angesehener Ausführungsform kann die Spritze 4 nach der Befüllung mit der als Medikament oder Wirkstoff transferierten Flüssigkeit auch zur Injektion der Flüssigkeit in einen Infusionsbeutel ("IV-Bag") verwendet werden. Dadurch kann der Wirkstoff oder das Medikament beispielsweise der eigentlichen Infusionsflüssigkeit beigemischt oder unmittelbar als Infusionsflüssigkeit bereitgestellt werden. Von dem Infusionsbeutel aus kann der Wirkstoff dann anschließend über ein intravenöses Applikationssystem appliziert werden.

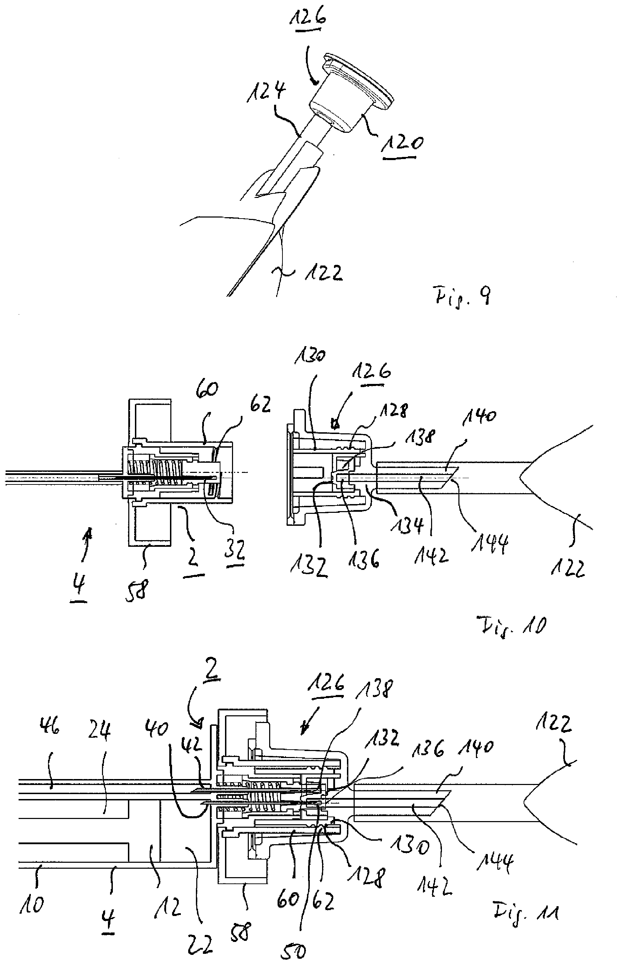

Um dies zu ermöglichen, ist ein als eigenständig erfinderisch angesehener Adapter 120 für einen Infusionsbeutel 122 vorgesehen, wie er gemeinsam mit dem Infusionsbeutel 122 in perspektivischer Ansicht in Fig. 9 und - jeweils im Längsschnitt gemeinsam mit dem Endbereich der Spritze 4 - in Fig. 10 vor und in Fig. 11 nach der Montage mit der Spritze 4 gezeigt ist.

Der Infusionsbeutel 122 gem. Fig. 9, auch als "IV-Bag" bezeichnet, ist zum Anschluss an ein intravenöses Applikationssystem vorgesehen, mit dem eine im Infusionsbeutel 122 vorgehaltene Flüssigkeit, beispielsweise ein Medikament oder auch eine isotonische Flüssigkeit oder dergleichen, einem Patienten als Infusion verabreicht werden kann. Der Infusionsbeutel 122 ist mit einem Anschlussstück 124 versehen, über das ein Mediensaustausch, insbesondere das Einbringen oder die Entnahme von Flüssigkeit in/aus dem Inneren des Infusionsbeutels 122 möglich ist. Um die Nutzung der Spritze 4 zum Flüssigkeitstransfer in den Infusionsbeutel 122 hinein (oder bedarfsweise auch aus diesem heraus) zu ermöglichen, ist der Adapter 120 vorgesehen, der in den Figs. 9 bis 11 in bereits an das Anschlussstück 124 des Infusionsbeutels anmontiertem Zustand gezeigt ist.

Der in den Figs. 10, 11 im Längsschnitt gezeigte Adapter 120 ist wie dargestellt zur Montage an die medizinische Spritze 4 unter Nutzung von deren erster Verbindungskomponente 2 ausgelegt. Dazu ist der Adapter 120 entsprechend mit einer zum Anschluss an die erste Verbindungskomponente 2 geeignet ausgestalteten weiteren Verbindungskomponente 126 versehen. Diese umfasst analog zu den vorstehend beschriebenen Varianten einen mit einem Gewinde 128 versehenen Verbindungsstutzen 130. Dieser ist dabei - ebenso wie die entsprechenden Komponenten der zweiten Verbindungskomponente 6 und die entsprechenden Komponenten der weiteren Verbindungskomponente 92 - an den Flanschmantel 60 der ersten Verbindungskomponente 2 und dessen Gewinde 62 angepasst ausgeführt, so dass diese Komponenten miteinander eine Luer-Verbindung bilden.

Innerhalb des Verbindungsstutzens 130 ist ein Kontaktelement 132 zur Herstellung einer medienseitigen Verbindung mit dem Nadelsystem 32 angeordnet. Das Kontaktelement 132 wird - in ähnlicher Ausgestaltung zum vorgenannten Kontaktelement 98 - im Wesentlichen durch einen Grundkörper 134 gebildet, der eine Kavität oder einen Hohlraum 136 zur Aufnahme der Nadelspitze 50 der ersten, für den Flüssigkeitstransfer vorgesehenen Hohlnadel 40 und eine weitere Kavität oder einen weiteren Hohlraum 138 zur Aufnahme der Nadelspitze 48 der zweiten, medienseitig mit dem Gaskanal 46 verbundenen Hohlnadel 42 aufweist. Ein gekapselter Gasaustausch ist in diesem Falle nicht vorgesehen, und dementsprechend ist der weitere Hohlraum 138 in der Art eines Sacklochs ausgeführt und mit keinen weiteren Medienkanälen verbunden.

Im Gegensatz dazu ist der Hohlraum 136 als Transferraum zur Weiterleitung der über die Hohlnadel 40 aus dem distalen Teilvolumen 22 der Spritze 4 eingespeisten Flüssigkeit ausgelegt und vorgesehen. Dazu geht der Hohlraum 136 in einen in einem Anschlussstutzen 140 oder einer Tülle in der Art einer axialen Zentralbohrung angeordneten Medienkanals 142, dessen freies Ende 144 medienseitig mit dem Innenraum des Infusionsbeutels 122 kommuniziert.

### Bezugszeichenliste

- 1: Flüssigkeitstransfersystem
- 2: erste Verbindungskomponente
- 4: Medizinische Spritze
- 6: zweite Verbindungskomponente
- 8: Container für medizinische Substanzen oder Arzneimittel
- 10: Hohlkörper
- 12: Kolben
- 14: Deckelplatte
- 16: Betätigungsstößel
- 18: freies Ende
- 20: Drückerplatte
- 22,24: Teilvolumen
- 26: distales Ende
- 30: Endplatte
- 32: Nadelsystem
- 34: Verbindungsstutzen
- 40,42: Hohlnadel
- 44: inneres Ende
- 46: Gaskanal
- 48,50: Nadelspitze
- 52: Nadelkappe
- 54: Rippe
- 56: Verbindungsmuffe
- 58: Betätigungsrand
- 60: Flanschmantel
- 62: Gewinde
- 70: Gewinde
- 72: Verbindungstutzen
- 74: Verschlussstopfen
- 76: Verschlusskappe
- 78: Einwegverschluss
- 80: Siegeldeckel
- 82: Federelement
- 84: Spiralfeder
- 86: Kappenkörper
- 88: Nadelschutzkörper
- 90: Spritzenadapter
- 92: Verbindungskomponente
- 94: Gewinde
- 96: Verbindungsstutzen
- 98: Kontaktelement
- 100: Grundkörper
- 102, 104: Hohlraum
- 106: Transfernadel
- 108: Hohlkörper
- 110: distales Ende
- 112: Nadelhalter
- 114: Hohlnadel
- 116: Lagermuffe
- 118: Nadelschutz
- 120: Adapter
- 122: Infusionsbeutel
- 124: Anschlussstück
- 126: Verbindungskomponente
- 128: Gewinde
- 130: Verbindungsstutzen
- 132: Kontaktelement
- 134: Grundkörper
- 136, 138: Hohlraum
- 140: Anschlussstutzen
- 142: Medienkanal
- 144: freies Ende

## Patentansprüche

1. Erste Verbindungskomponente (2) für eine medizinische Spritze (4) mit einem eine Anzahl von Hohlnadeln (40, 42) umfassenden, in einem rohrförmigen, von einem Flanschmantel (60) umgebenen Verbindungsstutzen (34) angeordneten Nadelsystem (32), und mit einer innerhalb des Verbindungsstutzens (34) befestigten, die distale(n) Spitze(n) (48, 50) der oder jeder Nadel (40, 42) des Nadelsystems (32) im unbelasteten Zustand dichtend umschließenden elastischen Nadelkappe (52), **dadurch gekennzeichnet, dass** der Flanschmantel (60) um das Nadelsystem (32) und um die dieses umschließende Nadelkappe (52) rotierbar ist und mit einem Gewinde (62) zum Anschluss an eine zweite Verbindungskomponente (6) versehen ist.

2. Erste Verbindungskomponente (2) nach Anspruch 1, deren Nadelsystem (32) als Doppelnadelsystem mit einer für einen Flüssigkeitstransfer vorgesehenen ersten Hohlnadel (40) und mit einer für einen Gastransfer vorgesehenen zweiten Hohlnadel (42) ausgeführt ist.

3. Spritze (4), die an ihrem distalen Ende (26) mit einer ersten Verbindungskomponente (34) nach einem der Ansprüche 1 bis 2 versehen ist.

4. Zweite Verbindungskomponente (6), insbesondere für einen Container (8) für medizinische Substanzen oder Arzneimittel oder für ein Transfersystem für medizinische Substanzen oder Arzneimittel, zum Anschluss an eine erste Verbindungskomponente (2) nach einem der Ansprüche 1 bis 2, mit einem mit einem zum Anschluss an dem Gewinde (62) des Flanschmantels (60) der ersten Verbindungskomponente (2) vorgesehenen Gewinde (70) versehenen Verbindungsstutzen (72), in dem ein aus einem Polypropylen bestehender Verschlusstopfen (74) angeordnet ist.

5. Container (8) für medizinische Substanzen oder Arzneimittel mit einer zweiten Verbindungskomponente (6) nach Anspruch 4.

6. Container (8) nach Anspruch 5, mit einer äußeren, die zweite Verbindungskomponente (6) umgebenden Kappe (76), die mit einem Einwegverschluss (78) versehen ist.

7. Flüssigkeitstransfersystem (1) zum Transfer von medizinischen Substanzen oder Arzneimitteln, umfassend eine erste Verbindungskomponente (2) nach einem der Ansprüche 1 bis 2 und, hinsichtlich der Dimensionierung und Ausführung der Verbindungstutzen (34, 72) an diese angepasst, eine zweite Verbindungskomponente (6).

## Claims

1. A first connecting component (2) for a medical syringe (4) with a needle system (32) comprising a number of hollow needles (40, 42) disposed in a tubular connecting piece (34) surrounded by a flanged sheath (60), and with an elastic needle cap (52) secured inside the connecting piece (34) which sealingly encloses the distal tip(s) (48, 50) of the or each needle (40, 42) of the needle system (32) in the unloaded state, **characterized in that** the flanged sheath (60) can be rotated about the needle system (32) and about the needle cap (52) enclosing it and is provided with a thread (62) for connecting to a second connecting component (6).

2. The connecting component (2) as claimed in claim 1, the needle system (32) of which being configured as a double needle system with a first hollow needle (40) provided for transferring a liquid and with a second hollow needle (42) provided for transferring a gas.

3. A syringe (4) which is provided at its distal end (26) with a first connecting component (34) as claimed in one of claims 1 to 2.

4. A second connecting component (6), in particular for a container (8) for medical substances or medicinal products or for a transfer system for medical substances or medicinal products, for connection to a first connecting component (2) as claimed in one of claims 1 to 2, with a connecting piece (72) provided with a thread (70) for connection to the thread (62) of the flanged sheath (60) of the first connecting component (2), in which a sealing plug (74) consisting of polypropylene is disposed.

5. A container (8) for medical substances or medicinal products, with a second connecting component (6) as claimed in claim 4.

6. The container (8) as claimed in claim 5, with an outer cap (76) surrounding the second connecting component (6) which is provided with a single-use closure (78).

7. A liquid transfer system (1) for transferring medical substances or medicinal products, comprising a first connecting component (2) as claimed in one of claims 1 to 2 and a second connecting component (6) which is adapted thereto in respect of the dimensions and configuration of the connecting piece (34, 72),

## Revendications

1. Premier composant (2) pour une seringue médicale (4) comprenant un système d'aiguilles (32) comprenant un nombre d'aiguilles creuses (40, 42), disposé dans une tubulure de liaison (34) tubulaire entourée par une enveloppe à bride (60), et comprenant un capuchon d'aiguilles (52) élastique fixé à l'intérieur de la tubulure de liaison (34), entourant de manière étanche la ou les pointe (s) distale(s) (48, 50) de la ou de chaque aiguille (40, 42) du système d'aiguilles (32) dans l'état non chargé, **caractérisé en ce que** l'enveloppe à bride (60) peut tourner autour du système d'aiguilles (32) et autour du capuchon d'aiguilles (52) entourant celui-ci et est doté d'un filetage (62) pour le raccordement à un second composant (6) de liaison.

2. Premier composant (2) selon la revendication 1, dont le système d'aiguilles (32) est conçu en tant que système d'aiguilles double avec une première aiguille creuse (40) prévue pour un transfert de liquide et avec une seconde aiguille creuse (42) prévue pour un transfert de gaz.

3. Seringue (4) qui est dotée à son extrémité distale (26) d'un premier composant de liaison (34) selon l'une des revendications 1 à 2.

4. Second composant (6), en particulier pour un récipient (8) pour des substances médicales ou des médicaments ou pour un système de transfert pour des substances médicales ou des médicaments, destiné au raccordement à un premier composant de liaison (2) selon l'une des revendications 1 à 2, comprenant une tubulure de liaison (72) dotée d'un filetage (70) prévu pour le raccordement sur le filetage (62) de l'enveloppe à bride (60) du premier composant de liaison (2), dans lequel est disposé un bouchon de fermeture (74) fabriqué en un polypropylène.

5. Récipient (8) pour des substances médicales ou des médicaments comprenant un second composant de liaison (6) selon la revendication 4.

6. Récipient (8) selon la revendication 5, comprenant un capuchon (76) extérieur entourant le second composant de liaison (6), qui est doté d'une fermeture à usage unique (78).

7. Système de transfert de liquide (1) pour le transfert de substances médicales ou de médicaments, comprenant un premier composant de liaison (2) selon l'une des revendications 1 à 2, et, au vu du dimensionnement et de la conception des tubulures (34, 72), adapté à celles-ci, un second composant (6).
